# FASCICULE DE BREVET EUROPEEN

(11) **EP 4 378 392 B1**
(45) Date de publication et mention de la délivrance du brevet: **24.12.2025**
(21) Numéro de dépôt: 23212515.3
(22) Date de dépôt: 28.11.2023
(51) Int. Cl.: A61B 5/378, A61B 5/38, A61B 5/00, A61B 5/16, G06F 3/01

(54) **DÉTERMINATION DE STIMULI OPTIMAUX POUR UNE INTERFACE À BASE DE POTENTIELS ÉVOQUÉS**
BESTIMMUNG OPTIMALER STIMULI FÜR EINE SCHNITTSTELLE AUF BASIS EVOZIERTER POTENTIALE
DETERMINING OPTIMAL STIMULI FOR AN INTERFACE BASED ON EVOKED POTENTIALS

(30) Priorité: 01.12.2022 FR 2212653
(43) Date de publication de la demande: 05.06.2024
(73) Titulaire: Orange, 92130 Issy-les-Moulineaux (FR)
(72) Inventeur: NICOL, Rozenn, 92326 CHATILLON CEDEX (FR); PLAPOUS, Cyril, 92326 CHATILLON CEDEX (FR); GUEHO, Lenaig, 92326 CHATILLON CEDEX (FR)
(74) Mandataire: Plasseraud IP

(56) Documents cités:
- EP-B1- 1 073 369
- WO-A1-2006/122349
- FERNANDEZ-PARRA J MANUEL ET AL: "Review and Application of Auditory Steady State Responses", 2018 IEEE-EMBS CONFERENCE ON BIOMEDICAL ENGINEERING AND SCIENCES (IECBES), IEEE, 3 December 2018 (2018-12-03), pages 661 - 666, XP033514179, DOI: 10.1109/IECBES.2018.8626643

## Description

### Domaine technique

La présente divulgation relève du domaine de la détection des potentiels évoqués par des interfaces neuronales directes (ou BCI ci-après, pour « Brain Computer Interface »).

### Technique antérieure

A titre d'exemple ci-après, les potentiels évoqués peuvent être auditifs, ou alternativement, visuels, tactiles, etc.

Un potentiel évoqué est un signal qui apparait dans les signaux électroencéphalogramme (EEG ci-après) lorsqu'un utilisateur est soumis à un stimulus. Par exemple dans l'approche SSAEP (pour « Steady-State Auditory Evoked Potential »), le stimulus est auditif et constitué d'un son ayant une fréquence porteuse et auquel on applique une modulation sinusoïdale en amplitude. Un équipement tel qu'un casque à capteurs de signaux EEG permet de mesurer les signaux EEG et de retrouver dans ces signaux la fréquence de la modulation.

Un exemple de stimulus utilisé pour une application SSAEP est un sinus à une fréquence audible par tous (par exemple 1000Hz) modulé par un second sinus, par exemple, à 37 Hz. Le sinus à 1000 Hz est appelé la porteuse et permet d'entendre le signal modulant à 37 Hz. Ce dernier constitue l'information d'intérêt.

En effet, il est possible de retrouver dans les aires auditives du cerveau les fréquences de stimulation entre environ 1 et 200 Hz. Néanmoins, à cette gamme de fréquences de 1 à 200 Hz, celles-ci sont basses et peuvent être difficiles à reproduire et/ou à entendre. C'est pourquoi une fréquence porteuse est utilisée comme vecteur, permettant à l'utilisateur d'entendre plus facilement ces fréquences. Un stimulus (sinus modulant à 37 Hz) peut alors générer un potentiel évoqué auditif mesurable dans le cerveau à la même fréquence de 37 Hz (plus les éventuelles harmoniques). Cette fréquence est mesurable dans les signaux EEG de la personne soumise au stimulus. On peut donc retrouver dans les signaux EEG de l'utilisateur la fréquence de la modulation à 37 Hz.

Cependant, il n'y a pas de consensus dans la communauté scientifique concernant les meilleurs stimuli, c'est-à-dire, ceux à même de déclencher les potentiels évoqués les plus puissants, et donc offrant la meilleure lisibilité dans les signaux EEG.

Il existe plusieurs façons différentes de construire les stimuli, les gammes de fréquences conseillées variant grandement d'une étude à l'autre. Une partie de l'explication réside sans doute dans le fait que les signaux EEG sont très variables d'une personne à l'autre et varient largement en fonction de beaucoup de paramètres (fatigue, contexte, concentration, etc.). Certaines fréquences peuvent donc être plus facilement détectables chez certaines personnes que d'autres. Cela peut aussi varier chez une même personne au cours du temps et selon certaines conditions (état émotionnel, perturbations extérieures, fatigue selon l'heure de la journée, etc.). Des systèmes et procédés connus sont décrits dans le document FERNANDEZ-PARRA J MANUEL ET AL: "Review and Application of Auditory Steady State Responses",2018 IEEE-EMBS CONFERENCE ON BIOMEDICAL ENGINEERING AND SCIENCES (IECBES), IEEE, 3 décembre 2018 (2018-12-03), pages 661-666, DOI: 10.1109/IECBES.2018.8626643.

### Résumé

La présente divulgation vient améliorer la situation.

Elle propose à cet effet une solution pour obtenir les meilleurs stimuli pour chaque personne et dans un contexte donné.

Selon un premier aspect, il est proposé un procédé de détermination d'au moins un signal d'interface convenant pour un utilisateur, cette interface étant du type fonctionnant par détection d'un potentiel évoqué dans un signal physiologique de l'utilisateur en réaction à une émission de signal d'interface destinée à l'utilisateur. Le procédé comportant, suite à l'émission d'au moins un signal d'interface, dit « signal d'interface courant » :
sélectionner au moins le signal d'interface courant comme signal d'interface convenant pour l'utilisateur lorsque l'émission du signal d'interface courant a provoqué une intensité d'un signal physiologique mesuré de l'utilisateur, supérieure à un seuil, le signal physiologique résultant d'une réaction de l'utilisateur à une émission du signal d'interface courant, comportant une première fréquence, porteuse, et une deuxième fréquence, de modulation.

Le signal physiologique précité peut être par exemple un signal d'électroencéphalogramme (ou « signal EEG » ci-après).

L'interface précitée peut être de type BCI pour « Brain Computer Interface » consistant à détecter dans le signal EEG recueilli chez un utilisateur de cette interface un signal appelé « potentiel évoqué » car présentant la même fréquence que la deuxième fréquence précitée, de modulation, du signal de stimulus présenté à l'utilisateur.

Un tel procédé permet alors de sélectionner les formes optimales des signaux de stimuli afin de maximiser l'intensité des potentiels évoqués qui sont générés. Une telle sélection des meilleurs signaux de stimulation pour une interface BCI présente l'avantage d'adapter les propriétés des stimuli aux différents utilisateurs et aux différentes conditions d'utilisation (état émotionnel, fatigue, perturbations extérieures). Un dispositif d'interface utilisant ces stimuli peut donc être plus robuste car personnalisé pour un utilisateur donné et à ses conditions d'utilisation. En effet, de tels dispositifs de commande mentale (ou « BCI »), de type réactif car l'émission de potentiels évoqués vient en réponse à des stimuli, reposent sur l'analyse de l'activité cérébrale (par détection de potentiels évoqués selon par exemple une méthode de type « Steady-State Auditory Evoked Potential » (ou « SSAEP »). Il est donc avantageux que les signaux occasionnant les plus forts potentiels évoqués soient ceux utilisés prioritairement par le dispositif d'interface.

Ainsi, on comprendra que le procédé peut comporter:
- émettre au moins un signal d'interface, dit « signal d'interface courant »,
- recevoir le signal physiologique résultant d'une réaction de l'utilisateur à l'émission du signal d'interface courant,
- sélectionner au moins le signal d'interface courant comme signal d'interface convenant pour l'utilisateur lorsque l'émission du signal d'interface courant a provoqué une intensité mesurée du signal physiologique de l'utilisateur, supérieure à un seuil,
le signal d'interface courant comportant une première fréquence, porteuse, et une deuxième fréquence, de modulation.

Dans une réalisation, le signal d'interface émis peut résulter d'une transformation du signal d'interface courant.

Par exemple, le procédé peut comporter:
- transformer le signal d'interface courant tant qu'aucune sélection de signal d'interface convenant pour l'utilisateur n'est (ou n'a pas pu être) effectuée.

Dans une réalisation, le procédé peut alors comporter :
- mesurer le signal physiologique de l'utilisateur, en réaction à l'émission du signal d'interface courant,
- transformer le signal courant, et
- répéter la mesure et la transformation une pluralité de fois, afin de retenir une pluralité de signaux d'interface convenant pour l'utilisateur et dont les émissions respectives ont provoqué une intensité du signal physiologique de l'utilisateur, supérieure à un seuil.

Dans une réalisation, ce seuil peut être fonction d'une moyenne des intensités successivement mesurées.

Ainsi, dans une telle réalisation, il est proposé une analyse statistique des signaux de stimulation relativement à la réaction qu'ils suscitent chez l'utilisateur.

Dans une réalisation, la transformation du signal courant est opérée par modification d'au moins une fréquence parmi les première et deuxième fréquences précitées, et/ou encore par modification d'au moins une amplitude associée à une fréquence parmi les première et deuxième fréquences précitées, relativement à une amplitude associée à l'autre fréquence parmi les première et deuxième fréquences.

Ces modifications peuvent être opérées par pas successifs jusqu'à atteindre des plages de fréquences satisfaisantes par exemple, pour un individu donné.

En variante toutefois, la transformation du signal courant est opérée par ajout de bruit aléatoire dans une représentation temps-fréquence du signal courant.

En outre, un signal d'interface qui peut être retenu peut comporter une première portion, bruitée, suivie dans le temps par au moins une deuxième portion, non bruitée, appelée « temps de pause ». Ce type de signal peut provoquer des potentiels évoqués à fort rapport signal à bruit.

Dans une réalisation, le signal d'interface convenant pour l'utilisateur est retenu par la mise en œuvre de filtres de corrélation inverse.

La méthode dite « des filtres de corrélation inverse » est une méthode psychophysique qui est habituellement utilisée pour étudier les processus cognitifs, sans connaissance a priori de l'impact d'un stimulus particulier sur le système cognitif d'un individu. On soumet des sujets à de tels stimuli et on observe les réponses de ces sujets en menant une analyse statistique des réponses associées à chaque stimulus afin de déduire des propriétés de processus sous-jacents. Ainsi, il est possible d'optimiser les stimuli à partir des réponses collectées.

Par exemple, si la transformation aléatoire d'un signal d'origine déforme la fréquence de modulation de ce signal en la faisant passer par exemple de 35 Hz à 37 Hz, et que ce signal transformé provoque un potentiel évoqué intense, alors cette fréquence de modulation à 37 Hz peut être celle d'une nouvelle génération de signaux à tester sur le même individu. On comprendra ainsi que du bruit ajouté aléatoirement peut permettre d'améliorer les signaux d'interface.

Dans une réalisation où les signaux d'interface retenus sont des signaux sonores, le procédé peut comporter en outre un stockage de données des signaux retenus comme convenant pour l'utilisateur (par exemple les première et deuxième fréquences et les amplitudes associées) en vue d'une restitution sonore de plusieurs de ces signaux par des haut-parleurs, simultanément.

Dans une telle réalisation, l'utilisateur peut écouter simultanément plusieurs signaux audios à des fréquences de modulation différentes. Par exemple, ces signaux peuvent être joués par des haut-parleurs respectifs, distants les uns des autres pour permettre à l'utilisateur de se concentrer sur l'un de ces signaux en particulier. En se concentrant sur l'un de ces signaux, son cerveau génère une onde périodique présentant la même fréquence que la fréquence de modulation du signal sur lequel il s'est concentré. Il est donc recueilli un potentiel évoqué pour ce signal. Si ce signal (qui génère ainsi un potentiel évoqué) est associé à une commande particulière (par exemple monter le volume d'un poste de télévision), alors la détection du potentiel évoqué correspondant va générer une commande correspondante (comme monter le volume sonore de ce poste de télévision). Si l'utilisateur s'était concentré sur un autre signal sonore (de fréquence de modulation différente par exemple), une autre commande aurait été exécutée (par exemple « changer de chaine de télévision », ou autre).

Une fréquence porteuse convenant pour un signal sonore d'une telle interface BCI est comprise par exemple entre 500 à 3500Hz. La fréquence de modulation peut quant à elle être comprise entre 1 et 100Hz, et plus particulièrement entre 20 et 80 Hz.

Il s'agit alors de plages de départ dans lesquelles peuvent être sélectionnées les fréquences de signaux initiaux d'interface qui peuvent être testés puis transformés pour modifier par exemple leur fréquence porteuse et/ou leur fréquence de modulation.

Pour ce qui est de l'évaluation de l'intensité du signal physiologique d'un utilisateur, la mesure de ce signal physiologique pour un utilisateur, en réaction à une émission de signal d'interface courant, peut comporter une recherche d'une fréquence dans le signal physiologique correspondant à la deuxième fréquence, de modulation du signal d'interface.

Plus particulièrement, il peut être déterminé « l'intensité du signal physiologique » de l'utilisateur par l'estimation d'un rapport signal à bruit, du signal physiologique mesuré sur l'utilisateur.

Ainsi, dans une réalisation, le procédé peut comporter:
- retenir au moins le signal d'interface convenant pour l'utilisateur, dont l'émission a provoqué une maximisation, à la fréquence de modulation, du rapport signal à bruit, du signal physiologique mesuré sur l'utilisateur.

Selon un autre aspect, il est proposé un programme informatique comportant des instructions pour la mise en œuvre de tout ou partie d'un procédé tel que défini dans les présentes lorsque ce programme est exécuté par un processeur. Selon un autre aspect, il est proposé un support d'enregistrement non transitoire, lisible par un ordinateur, sur lequel est enregistré un tel programme.

Selon un autre aspect, il est proposé un dispositif comportant un circuit de traitement pour la mise en œuvre du procédé ci-avant.

### Brève description des dessins

D'autres caractéristiques, détails et avantages apparaîtront à la lecture de la description détaillée ci-après, et à l'analyse des dessins annexés, sur lesquels :
**Fig. 1**

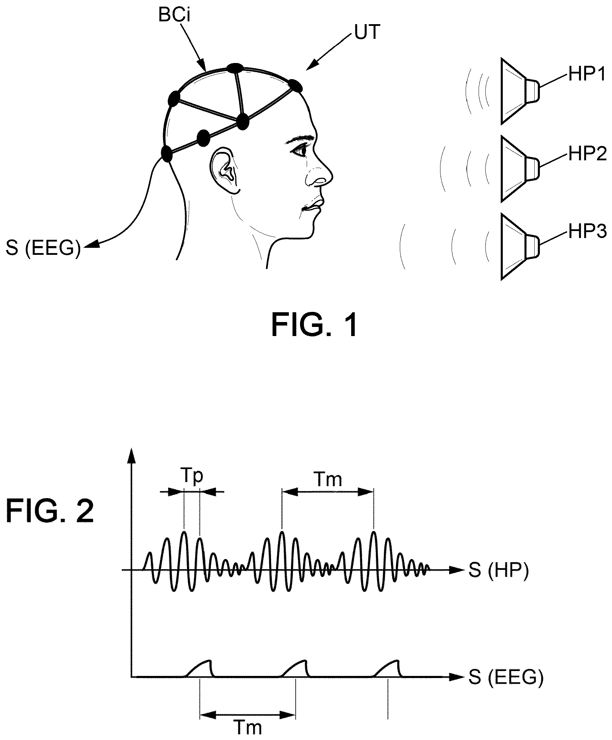

   [Fig. 1] montre un exemple d'application du procédé ci-avant à une interface de type BCI selon un mode de réalisation.
**Fig. 2**
   [Fig. 2] montre un exemple de signal généré avec une fréquence porteuse fp et une fréquence de modulation fm (au-dessus) et (en-dessous) un signal de potentiel évoqué détecté chez un utilisateur avec une fréquence identique à celle de la fréquence de modulation fm.
**Fig. 3**

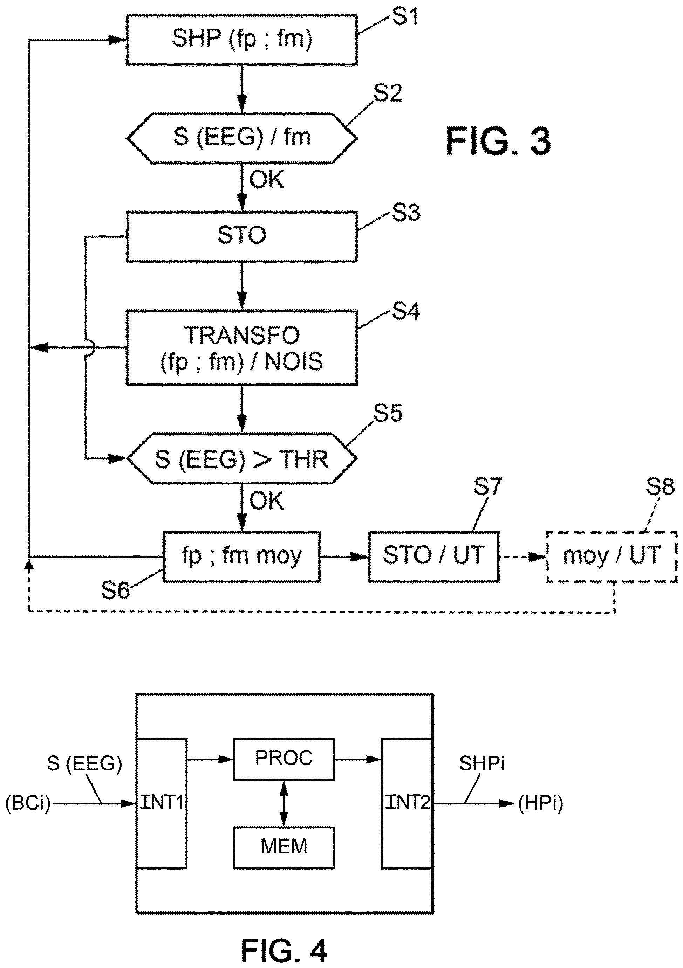

   [Fig. 3] montre un exemple de procédé au sens de la présente description, selon un mode de réalisation.
**Fig. 4**
   [Fig. 4] montre un exemple de dispositif au sens de la présente description, selon un mode de réalisation.

### Description des modes de réalisation

On se réfère tout d'abord à la figure 1 pour rappeler le principe d'une interface BCI (pour « Brain Computer Interface »). Un utilisateur UT porte un casque à capteurs de signaux d'électroencéphalogramme (ou « signaux EEG » ci-après), ce casque pouvant faire partie d'une interface générale BCI. Différents stimuli (sonores, visuels, ou autres), ayant des fréquences de modulation respectives, sont délivrés à l'utilisateur, lequel se concentre sur l'un des stimuli. Il peut être alors mesuré dans ses signaux EEG la même fréquence de modulation que celle du stimulus sur lequel l'utilisateur s'est concentré. Dans l'exemple d'un stimulus délivré par une source lumineuse, cette dernière peut clignoter à la fréquence de modulation précitée. Dans le cas d'un signal sonore, le signal peut comporter une modulation sinusoïdale ou peut être constitué de bips successifs à la fréquence de modulation précitée. Une analyse des signaux EEG de l'utilisateur révèle la présence d'une fréquence d'onde correspondant à la fréquence de modulation précitée. Dès lors, il est possible de concevoir une interface BCI, selon laquelle plusieurs stimuli issus de sources respectives HP1, HP2, HP3, ayant des fréquences de modulation différentes et représentant par exemple des consignes respectives différentes pour piloter une machine (par exemple « tourner à gauche », « tourner à droite », « freiner »), sont présentés simultanément à un utilisateur et ce dernier se concentre sur l'un d'eux de sorte que ses signaux EEG révèle la fréquence de l'un des stimuli HPi et la fonction associée à ce stimulus est alors exécutée par la machine.

Dans l'exemple de la figure 1, les stimuli sont délivrés par des haut-parleurs respectifs HP1, HP2, HP3... et l'utilisateur se concentre sur l'une de ces sources sonores. Ainsi, on décrit ci-après une réalisation dans laquelle le signal d'interface (stimulus appliqué à l'utilisateur UT) est par exemple un signal audio pour relever un signal physiologique de type SSAEP (pour « Steady-State Auditory Evoked Potential »). Le stimulus est donc auditif et constitué d'un son à une fréquence de porteuse fp auquel on applique une modulation sinusoïdale ou en créneau, à une fréquence de modulation fm. Les stimuli sont généralement construits avec une fréquence porteuse (correspondant à la période Tp de la figure 2), et modulés en amplitude, en puissance ou en énergie, par un signal ayant une deuxième fréquence (correspondant à la période Tm de la figure 2). Si le signal de stimulation est efficace, le signal EEG mesuré par le casque de l'interface BCI présente une fréquence correspondant à la période Tm de la figure 2.

Dans le cas d'un stimulus visuel, une source lumineuse peut avoir une couleur particulière, correspondant ainsi à une longueur d'onde LO associée à une fréquence porteuse fp par une relation du type LO = c / fp, où c est la vitesse de la lumière. Certains utilisateurs peuvent être plus sensibles à certaines couleurs que d'autres pour retenir leur attention. Ainsi, le choix de la couleur (et donc de la fréquence de la porteuse fp) peut être important, notamment à certains moments de la journée pour un même utilisateur, pour recueillir efficacement des signaux EEG révélant un potentiel évoqué exploitable (i.e. ayant une fréquence correspondant à la fréquence de modulation ou de « clignotement » de la source lumineuse). Par exemple, une longueur d'onde de 0,5µm peut être utilisée pour du bleu comme couleur de stimuli visuels chez certains sujets, ou une longueur d'onde de 0,55µm pour du vert auxquels certains utilisateurs sont plus sensibles, ou encore 0,65µm pour du rouge, couleur à laquelle d'autres utilisateurs sont encore plus sensibles, etc.

On comprend ainsi qu'une subjectivité qui est personnelle à chaque utilisateur fait en sorte que certains stimuli sont plus facilement perçus et peuvent être à l'origine de potentiels évoqués exploitables pour concevoir une interface BCI robuste pour un utilisateur donné.

On présente ci-après en référence à la figure 3 un procédé pour sélectionner les stimuli les plus efficaces pour un utilisateur donné. A l'étape S1, on génère un signal de fréquence de porteuse fp avec une modulation de fréquence fm, que l'on applique sous forme de stimulus à un utilisateur. A l'étape S2, on recueille le signal EEG de l'utilisateur et l'on cherche en particulier à détecter dans ce signal S(EEG) une fréquence correspondant à la fréquence de modulation fm. Les signaux de stimulus SHP ayant réussi par exemple à générer un potentiel évoqué à l'étape S2 sont stockés en mémoire à l'étape S3. A partir de ces signaux de stimulus, on peut chercher, à l'étape S4, à transformer progressivement des versions successives de ces signaux :
- en procédant par exemple à une modification par pas successifs de la fréquence de modulation fm et/ou de la fréquence porteuse fp, et/ou de leurs amplitudes respectivement associées pour construire le signal de stimulus SHP, selon une première forme de réalisation, et/ou
- en appliquant des variations aléatoires dans le temps, de bruit, à une représentation temps-fréquence des signaux de stimulus stockés en mémoire à l'étape S3, selon une deuxième forme de réalisation,
puis en mettant en œuvre une technique de « filtrage par corrélation inverse », décrite en détail plus loin.

Ensuite, les transformations de stimuli ayant occasionné des potentiels évoqués sont à leur tour stockées en mémoire (boucle à nouveau sur les étapes S1à S3 de la figure 1). A l'étape S5, on peut retenir alors les versions transformées des stimuli qui ont occasionné des potentiels évoqués avec un rapport signal à bruit (signal EEG à la fréquence de modulation fm) supérieur à un seuil THR. Ce seuil THR peut être fixe ou au contraire être estimé en fonction de la moyenne des rapports signal à bruit. Par exemple, on peut retenir tous les stimuli dont le rapport signal à bruit est supérieur à la moyenne des rapports signal à bruit (éventuellement plus un ou plusieurs écarts-types par exemple). A l'étape S6, s'il apparait quelques fréquences de modulation fm et/ou quelques fréquences porteuses fp dont les signaux de stimulus ont fourni des résultats particulièrement satisfaisants au test S5, il peut être construit des signaux de stimulus avec des fréquences fm et/ou fp moyennées parmi ces « fréquences satisfaisantes » pour tester à nouveau de nouvelles versions de stimuli issues de ces fréquences moyennes (boucle sur les étapes S1 à S5). Notamment dans le cas de l'ajout d'un bruit à variation temporelle aléatoire, il est possible de créer une modulation par l'ajout de ce bruit qui a pour effet de modifier aléatoirement la fréquence de modulation, ou même la fréquence porteuse. Dans ce cas, les nouvelles versions testées peuvent avoir ces fréquences de modulation et/ou de porteuse modifiées. Finalement, les meilleurs signaux de stimulus sont stockés en mémoire à l'étape S7 (fréquences fm et fp, amplitudes associées, éventuellement une forme de bruit aléatoire modifiant le signal, etc.). Ces signaux « satisfaisants » stockés à l'étape S7 peuvent correspondre ainsi à des stimuli efficaces pour générer des potentiels évoqués.

Il est possible aussi de stocker à l'étape S7 des signaux ayant des parties bruitées, avec éventuellement des « temps de pause » entre les parties bruitées qui correspondent à du signal non bruité.

A l'étape S7, les tests réalisés aux étapes S1 à S6 sur un utilisateur donné UT, permettent ainsi d'obtenir des signaux de stimulus satisfaisants pour une interface BCI qu'utiliserait cet utilisateur. Il a été observé néanmoins certaines tendances générales, au moins parmi des populations d'individus, de sorte que les signaux propres à un utilisateur UT pourraient convenir plus globalement à toute une catégorie d'utilisateurs. Ainsi, à l'étape S8, il est possible de moyenner des signaux « satisfaisants » d'après les tests réalisés sur plusieurs utilisateurs différents mais par exemple appartenant à une même catégorie (telle qu'une tranche d'âge, une activité professionnelle ou autre).Ainsi, il est possible d'affiner encore l'individualisation des signaux de stimuli propres à un utilisateur, une fois que sa catégorie a été identifiée à l'étape S8 en recommençant les étapes d'optimisation de ces signaux (comme illustré par la flèche en traits pointillés issue de l'étape S8 de la figure 3). Ainsi, on retiendra qu'à l'issue de l'étape S7, les signaux obtenus pour un utilisateur peuvent être utilisés pour l'interface BCI de cet utilisateur, et, pour aller plus loin (dans la réalisation en traits pointillés de l'étape S8 de la figure 3), ces signaux testés sur différents utilisateurs peuvent être moyennés pour être des signaux standards à partir desquels des optimisations individuelles peuvent être poursuivies par chaque utilisateur (une fois sa catégorie identifiée).

Pour des signaux audios par exemple, il a été observé en effet qu'une gamme de fréquences fp de la porteuse entre 500 et 3500 Hz était prometteuse pour obtenir rapidement des potentiels évoqués pour un grand nombre d'utilisateurs. La fréquence de modulation fm est plus petite en principe, par exemple dans une gamme de 1 à 100 Hz. Il a été observé en particulier qu'entre 20 et 80 Hz, les potentiels évoqués pouvaient être détectés pour un grand nombre d'utilisateurs, et les utilisateurs plus jeunes pourraient être plus sensibles aux hautes fréquences (avoisinant 60 Hz jusqu'à 80 Hz).

Le signal de porteuse peut être modulé avec cette fréquence plus basse fm, par une fonction de type sinus ou encore par un créneau. Il est aussi possible d'introduire des silences dans le signal de porteuse pour générer une répétition de forme « signal - silence » à une fréquence fm correspondant à celle de la modulation précitée. A l'étape S4, il est également possible de modifier l'amplitude de signal de porteuse comme l'amplitude de la modulation.

Il est généré ainsi des stimuli sonores successivement avec une modification d'un de ces paramètres (fréquence / amplitude ; porteuse / modulation ; fonction sinus / créneau) ou de plusieurs de ces paramètres. Des potentiels évoqués qui peuvent éventuellement advenir en réaction de l'utilisateur à ces stimuli sont détectés dans les signaux EEG de l'utilisateur. Les paramètres précités d'amplitudes et fréquences respectives peuvent être stockés en mémoire à l'étape S3, en correspondance d'une mesure d'un rapport signal à bruit des signaux EEG à cette fréquence de modulation.

La manière de faire varier ces paramètres précités peut être par pas progressifs (par exemple augmentation par pas de la fréquence de modulation à partir de 20 Hz pour identifier quelques fréquences de modulation qui conviennent particulièrement à l'utilisateur car les potentiels évoqués détectés présentent un fort rapport signal à bruit à ces fréquences). Alternativement, la manière de faire varier ces paramètres peut être aléatoire ou suivre une loi de probabilité prédéfinie.

En particulier, une forme de réalisation possible met en œuvre une « méthode de corrélation inverse ». Cette technique s'appuie sur la subjectivité des tests. La transformation du stimulus proposé à l'utilisateur est aléatoire et la réaction de l'utilisateur peut être inattendue. Par exemple, partant d'une fréquence de modulation de 30 Hz, l'utilisateur peut réagir moins bien à 35 Hz qu'à 30 Hz, mais mieux à 40 Hz qu'à 30 Hz, et ce de façon reproductible. Ceci peut s'expliquer par des processus cognitifs propres à l'utilisateur.

Partant de ce constat, il est appliqué par exemple une transformation temps-fréquence aux signaux de stimuli et il est ajouté à cette représentation temps-fréquence un bruit aléatoire (variant aléatoirement dans le temps) pour générer de nouveaux stimuli à soumettre à l'utilisateur. Une telle transformation par ajout de bruit aléatoire peut modifier par exemple la fréquence de la porteuse et/ou de la modulation. Si l'utilisateur y réagit avec une forte intensité de potentiel évoqué (mesurée par son rapport signal à bruit au test S5), alors les caractéristiques de signal d'un tel stimulus sont stockées en mémoire à l'étape S7 pour faire partie des signaux d'interface convenant pour l'utilisateur.

Ainsi, au lieu de faire varier de façon fine les paramètres identifiés (fréquence de la porteuse, fréquence du signal modulant, amplitude de la porteuse, amplitude du signal modulant, etc.), comme décrit dans la première forme de réalisation, il est proposé, dans cette deuxième forme de réalisation, de bruiter les stimuli à l'étape S4 afin de créer à l'étape S6 un nouvel ensemble de stimuli à évaluer (boucle vers l'étape S1 à nouveau).

Chaque sujet UT écoute ces signaux. De façon synchrone, ses signaux EEG sont enregistrés. Il est alors possible de déterminer quels signaux ont déclenché les potentiels évoqués les plus puissants. On peut alors identifier les stimuli optimaux pour un utilisateur donné.

Dans une réalisation, il est proposé alors de construire les meilleurs stimuli grâce à l'approche dite de « corrélation inverse ». La méthode des filtres de corrélation inverse est une méthode psychophysique pour étudier les processus cognitifs. Par exemple dans d'autres techniques, l'image d'un visage très bruitée peut susciter différentes émotions chez différents sujets (par exemple de la peur pour certains individus car le visage est perçu avec une expression de colère, ou de la joie car le visage est perçu par d'autres individus comme souriant, par exemple). La corrélation inverse utilise ainsi la réponse de l'utilisateur, très personnelle, à un bruitage d'un stimulus. Le système cognitif est traité ici comme une boîte noire. On soumet des sujets à ces stimuli et on observe leurs réponses. L'analyse statistique des réponses associées à chaque stimulus permet de déduire les propriétés des processus sous-jacents. Ainsi, il est possible d'optimiser les stimuli à partir des réponses collectées. Dans un premier temps, plusieurs stimuli sont générés à partir d'un stimulus donné. Le stimulus donné et les stimuli constituant des variants du stimulus donné constitue un ensemble de stimuli à tester. Ensuite, au moins certains des stimuli de cet ensemble de stimuli à tester sont reproduits successivement.

Ainsi, les stimuli audios sont considérés ici comme une image en les transformant dans leur représentation temps-fréquence. La représentation de leur spectrogramme peut être obtenue en découpant le signal en trames et en transformant chaque trame en une représentation fréquentielle, pour obtenir une image temps-fréquence représentant chaque stimulus.

Ensuite, pour chaque stimulus reproduit, un potentiel évoqué est détecté et une analyse statistique notamment par corrélation inverse du potentiel évoqué détecté et du stimulus reproduit ayant déclenché le potentiel évoqué détecté est effectuée. Notamment, pour chaque potentiel évoqué détecté, la puissance (ou le RSB comme vu précédemment) du signal du potentiel évoqué est mesurée.

Des stimuli sont collectés/sélectionnés parmi les stimuli reproduits en fonction du résultat de l'analyse statistique, notamment en fonction de l'intensité de signal des potentiels évoqués détectés (les stimuli correspondants, par exemple, aux potentiels évoqués détectés dont les signaux sont les plus intenses ou aux potentiels évoqués détectés dont l'intensité est supérieure à un seuil de puissance donné).

Eventuellement, les stimuli collectés peuvent être moyennés, comme décrit précédemment. En particulier, afin de non seulement optimiser les stimuli mais aussi de les personnaliser, la sélection et/ou le moyennage sont effectués en fonction de potentiels évoqués détectés pour un utilisateur donné.

En pratique, on peut utiliser plusieurs stimuli avec différentes porteuses et différentes fréquences de modulation. Chaque stimulus ainsi obtenu est ensuite transformé en image en utilisant une représentation temps-fréquence (spectrogramme). Chaque spectrogramme est perturbé par un bruit aléatoire (l'image étant bruitée). Le spectrogramme bruité résultant est ensuite transformé pour redevenir un signal audio.

Ces stimuli audios perturbés peuvent servir à nouveau, éventuellement une pluralité de fois, à nourrir un test subjectif basé sur le principe de la corrélation inverse, comme décrit ci-dessus. Différents sujets peuvent écouter ces signaux. De façon synchrone, leurs signaux EEG sont enregistrés. Il est alors possible de déterminer les signaux qui ont déclenché les potentiels évoqués les plus puissants. Par « plus puissants », il est fait référence au rapport signal utile à bruit (RSB) du signal EEG. On cherche alors à déterminer les stimuli maximisant le RSB dans les fréquences mesurées dans les signaux EEG récoltés, en particulier à la fréquence de la modulation. Par exemple, on peut définir un seuil de RSB à partir duquel les potentiels évoqués sont considérés comme robustes et valides pour être détectés. L'ensemble des stimuli répondant à ce critère est collecté. Les formes d'onde de ces stimuli peuvent ensuite être moyennées individuellement pour chaque sujet afin d'obtenir des stimuli optimaux personnalisés (i.e. individuels) pour un sujet donné.

Ainsi, la technique ci-avant réunit les avantages suivants :
- Sélection automatique des propriétés des stimuli donnant les meilleures performances, notamment en termes de détectabilité et de temps de réponse,
- Adaptation automatique d'une interface BCI à différents utilisateurs,
- Robustesse de la détection à la modification des conditions d'utilisation (état émotionnel, fatigue, perturbations extérieures).

On a illustré sur la figure 4 une réalisation possible d'un dispositif pour mettre en œuvre la technique ci-avant. Le dispositif peut comporter typiquement :
- une interface INT2 pour émettre des signaux de stimulation SHPi destinés par exemple à alimenter des haut-parleurs (ou des sources lumineuses),
- une autre interface INT1 pour recevoir les signaux EEG issus des capteurs du casque d'interface BCI,
- un processeur PROC pour réaliser les tests de la figure 3 notamment et identifier les signaux SHPi provoquant les potentiels évoqués les plus intenses dans les signaux S(EEG), et
- une mémoire MEM stockant les données de ces signaux SHPi pour la mise en œuvre de l'étape S7 de la figure 3 (ou de l'étape S8 si un moyennage est effectué sur plusieurs individus).

En particulier, la mémoire MEM peut être un bloc mémoires stockant en outre les données d'instructions d'un programme informatique, le processeur PROC coopérant avec la mémoire MEM pour lire ces données et exécuter ces instructions.

La technique décrite ci-avant peut trouver de nombreuses applications utilisant les interfaces BCI de type « réactives » (réaction à des stimuli), pour des personnes paralysées ou aveugles, ou encore pour une commande « par la pensée » à l'aide d'un casque d'interface BCI (notamment dans le cadre d'une commande de maison connectée avec des commandes telles que « allumer la télévision », « changer de chaîne », « allumer la lumière », « éteindre le chauffage », etc.).

Il convient de noter que la figure 4 peut en outre représenter les éléments matériels (hardware) d'une telle interface BCI. Par exemple, les haut-parleurs HPi de l'interface BCI peuvent correspondre aux oreillettes d'un casque en restitution stéréophonique et une partie au moins des signaux retenus en mémoire à l'étape S7 peuvent être joués simultanément par les oreillettes du casque à des « positions » stéréos respectives.

## Revendications

1. Procédé de détermination d'au moins un signal d'interface (BCI) convenant pour un utilisateur, ladite interface étant du type fonctionnant par détection d'un potentiel évoqué dans un signal physiologique (EEG) de l'utilisateur (UT) en réaction à une émission de signal d'interface destinée à l'utilisateur, le procédé comportant, suite à l'émission d'au moins un signal d'interface, dit signal d'interface courant :
sélectionner au moins le signal d'interface courant comme signal d'interface convenant pour l'utilisateur lorsque l'émission du signal d'interface courant a provoqué une intensité d'un signal physiologique mesuré (EEG) de l'utilisateur, supérieure à un seuil, le signal physiologique résultant d'une réaction de l'utilisateur à une émission du signal d'interface courant, comportant une première fréquence, porteuse, et une deuxième fréquence, de modulation.

2. Procédé selon la revendication 1, dans lequel le signal d'interface émis résulte d'une transformation du signal d'interface courant.

3. Procédé selon l'une quelconque des revendications précédentes, comportant :
- transformer le signal d'interface courant tant qu'aucune sélection de signal d'interface convenant pour l'utilisateur n'est effectuée.

4. Procédé selon l'une quelconque des revendications précédentes, comportant :
- mesurer le signal physiologique (EEG) de l'utilisateur, en réaction à l'émission du signal d'interface courant,
- transformer le signal courant, et
- répéter la mesure et la transformation une pluralité de fois, afin de retenir une pluralité de signaux d'interface convenant pour l'utilisateur et dont les émissions respectives ont provoqué une intensité du signal physiologique (EEG) de l'utilisateur, supérieure à un seuil.

5. Procédé selon la revendication 4, dans lequel ledit seuil est fonction d'une moyenne des intensités successivement mesurées.

6. Procédé selon l'une des revendications 2 à 5, dans lequel la transformation du signal d'interface courant est opérée par modification d'au moins une fréquence parmi les première et deuxième fréquences.

7. Procédé selon l'une des revendications 2 à 6, dans lequel la transformation du signal courant est opérée par modification d'au moins une amplitude associée à une fréquence parmi les première et deuxième fréquences, relativement à une amplitude associée à l'autre fréquence parmi les première et deuxième fréquences.

8. Procédé selon l'une des revendications 2 à 7, dans lequel la transformation du signal courant est opérée par ajout de bruit aléatoire dans une représentation temps-fréquence du signal courant.

9. Procédé selon l'une des revendications précédentes, dans lequel le signal retenu comporte au moins une première portion, bruitée, suivie dans le temps par une deuxième portion, non bruitée.

10. Procédé selon l'une des revendications précédentes, dans lequel ledit au moins un signal d'interface convenant pour l'utilisateur est retenu par mise en œuvre de filtres de corrélation inverse.

11. Procédé selon l'une quelconque des revendications précédentes, dans lequel les signaux d'interface sont des signaux sonores, et le procédé comporte en outre un stockage de données des signaux retenus comme convenant pour l'utilisateur en vue d'une restitution sonore, par des haut-parleurs, d'une partie au moins desdits signaux retenus, simultanément.

12. Procédé selon l'une des revendications précédentes, comportant une mesure du signal physiologique (EEG) de l'utilisateur, en réaction à une émission de signal d'interface courant, en recherchant une fréquence dans le signal physiologique (EEG) correspondant à la deuxième fréquence.

13. Procédé selon l'une des revendications précédentes, dans lequel l'intensité du signal physiologique (EEG) de l'utilisateur est déterminée par estimation d'un rapport signal à bruit, du signal physiologique (EEG) mesuré sur l'utilisateur.

14. Procédé selon les revendications 12 et 13, prises en combinaison, comportant :
- retenir au moins le signal d'interface convenant pour l'utilisateur, dont l'émission a provoqué une maximisation, à la fréquence de modulation, du rapport signal à bruit, du signal physiologique (EEG) mesuré sur l'utilisateur.

15. Programme informatique comportant des instructions pour la mise en œuvre du procédé selon l'une des revendications précédentes, lorsque ce programme est exécuté par un circuit de traitement.

16. Dispositif comportant un circuit de traitement pour la mise en œuvre du procédé selon l'une des revendications 1 à 14.

## Patentansprüche

1. Verfahren zur Bestimmung mindestens eines für einen Benutzer geeigneten Schnittstellensignals (BCI), wobei die Schnittstelle von der Art ist, die durch das Erkennen eines Potenzials funktioniert, das in einem physiologischen Signal (EEG) des Benutzers (UT) als Reaktion auf eine für den Benutzer bestimmte Schnittstellensignalaussendung evoziert wird, wobei das Verfahren im Anschluss an das Aussenden mindestens eines Schnittstellensignals, als aktuelles Schnittstellensignal bezeichnet, Folgendes umfasst:
Auswählen mindestens des aktuellen Schnittstellensignals als ein für den Benutzer geeignetes Schnittstellensignal, wenn die Aussendung des aktuellen Schnittstellensignals eine Stärke eines gemessenen physiologischen Signals (EEG) des Benutzers bewirkt hat, die größer als eine Schwelle ist, wobei das physiologische Signal aus einer Reaktion des Benutzers auf eine Aussendung des aktuellen Schnittstellensignals resultiert, das eine erste Trägerfrequenz und eine zweite Modulationsfrequenz umfasst.

2. Verfahren nach Anspruch 1, wobei das ausgesendete Schnittstellensignal aus einer Umwandlung des aktuellen Schnittstellensignals resultiert.

3. Verfahren nach einem der vorhergehenden Ansprüche, umfassend:
- Umwandeln des aktuellen Schnittstellensignals, solange keine Auswahl eines für den Benutzer geeigneten Schnittstellensignals getroffen wird.

4. Verfahren nach einem der vorhergehenden Ansprüche, umfassend:
- Messen des physiologischen Signals (EEG) des Benutzers als Reaktion auf die Aussendung des aktuellen Schnittstellensignals,
- Umwandeln des aktuellen Signals und
- Wiederholen der Messung und der Umwandlung mehrere Male, um eine Vielzahl von für den Benutzer geeigneten Schnittstellensignalen zu wählen, deren jeweilige Aussendungen eine Stärke des physiologischen Signals (EEG) des Benutzers bewirkt haben, die höher als eine Schwelle ist.

5. Verfahren nach Anspruch 4, wobei die Schwelle von einem Mittelwert der aufeinanderfolgend gemessenen Stärken abhängt.

6. Verfahren nach einem der Ansprüche 2 bis 5, wobei die Umwandlung des aktuellen Schnittstellensignals durch Änderung mindestens einer Frequenz von der ersten und der zweiten Frequenz umgesetzt wird.

7. Verfahren nach einem der Ansprüche 2 bis 6, wobei die Umwandlung des aktuellen Signals durch Änderung mindestens einer Amplitude, die mit einer Frequenz von der ersten und der zweiten Frequenz assoziiert ist, relativ zu einer Amplitude, die mit der anderen Frequenz von der ersten und der zweiten Frequenz assoziiert ist, umgesetzt wird.

8. Verfahren nach einem der Ansprüche 2 bis 7, wobei die Umwandlung des aktuellen Signals durch das Hinzufügen von Zufallsrauschen zu einer Zeit-Frequenz-Darstellung des aktuellen Signals umgesetzt wird.

9. Verfahren nach einem der vorhergehenden Ansprüche, wobei das gewählte Signal mindestens einen ersten, verrauschten, Abschnitt umfasst, zeitlich gefolgt von einem zweiten, nicht verrauschten, Abschnitt.

10. Verfahren nach einem der vorhergehenden Ansprüche, wobei das mindestens eine für den Benutzer geeignete Schnittstellensignal durch Implementierung von inversen Korrelationsfiltern gewählt wird.

11. Verfahren nach einem der vorhergehenden Ansprüche, wobei die Schnittstellensignale Tonsignale sind und das Verfahren ferner das Speichern von Daten der für den Benutzer als geeignet gewählten Signale zwecks einer simultanen Tonwiedergabe, über Lautsprecher, mindestens eines Teils der gewählten Signale umfasst.

12. Verfahren nach einem der vorhergehenden Ansprüche, umfassend eine Messung des physiologischen Signals (EEG) des Benutzers als Reaktion auf eine Aussendung eines aktuellen Schnittstellensignals, wobei nach einer Frequenz im physiologischen Signal (EEG) gesucht wird, die der zweiten Frequenz entspricht.

13. Verfahren nach einem der vorhergehenden Ansprüche, wobei die Stärke des physiologischen Signals (EEG) des Benutzers durch Ermittlung eines Signal-Rausch-Verhältnisses des an dem Benutzer gemessenen physiologischen Signals (EEG) bestimmt wird.

14. Verfahren nach den Ansprüchen 12 und 13 in Kombination, umfassend:
- Wählen mindestens des für den Benutzer geeigneten Schnittstellensignals, dessen Aussendung eine Maximierung, bei der Modulationsfrequenz, des Signal-Rausch-Verhältnisses des an dem Benutzer gemessenen physiologischen Signals (EEG) bewirkt hat.

15. Computerprogramm, das Anweisungen umfasst, um das Verfahren nach einem der vorhergehenden Ansprüche zu implementieren, wenn dieses Programm durch eine Verarbeitungsschaltung ausgeführt wird.

16. Vorrichtung, die eine Verarbeitungsschaltung zur Implementierung des Verfahrens nach einem der Ansprüche 1 bis 14 umfasst.

## Claims

1. Method for determining at least one interface (BCI) signal appropriate to a user, said interface being of the type that operates by detecting a potential produced in a physiological signal (EEG) of the user (UT) in response to the output of an interface signal to the user, the method comprising, following output of at least one interface signal, known as the current interface signal:
selecting at least the current interface signal as the interface signal appropriate to the user when output of the current interface signal has caused the intensity of a measured physiological signal (EEG) of the user to exceed a threshold, the physiological signal resulting from a response of the user to output of the current interface signal, comprising a first frequency, carrier frequency, and a second frequency, modulation frequency.

2. Method according to Claim 1, wherein the interface signal that is output results from a transformation of the current interface signal.

3. Method according to either one of the preceding claims, comprising:
- transforming the current interface signal as long as no interface signal appropriate to the user is selected.

4. Method according to any one of the preceding claims, comprising:
- measuring the physiological signal (EEG) of the user in response to output of the current interface signal,
- transforming the current signal, and
- repeating the measurement and transformation a plurality of times in order to retain a plurality of interface signals appropriate to the user that, when output, have each caused the intensity of the physiological signal (EEG) of the user to exceed a threshold.

5. Method according to Claim 4, wherein said threshold is dependent on an average of the successively measured intensities.

6. Method according to one of Claims 2 to 5, wherein the current interface signal is transformed by changing at least one of the first and second frequencies.

7. Method according to one of Claims 2 to 6, wherein the current signal is transformed by changing at least one amplitude associated with one of the first and second frequencies relative to an amplitude associated with the other of the first and second frequencies.

8. Method according to one of Claims 2 to 7, wherein the current signal is transformed by adding random noise to a time-frequency representation of the current signal.

9. Method according to one of the preceding claims, wherein the retained signal comprises at least a first, noisy portion followed in time by a second, non-noisy portion.

10. Method according to one of the preceding claims, wherein said at least one interface signal appropriate to the user is retained by using inverse correlation filters.

11. Method according to any one of the preceding claims, wherein the interface signals are sound signals, and the method further comprises storing data of the retained signals as appropriate to the user for audio reproduction, by loudspeakers, of at least some of said retained signals simultaneously.

12. Method according to one of the preceding claims, comprising measurement of the physiological signal (EEG) of the user in response to the output of a current interface signal by looking for a frequency in the physiological signal (EEG) that matches the second frequency.

13. Method according to one of the preceding claims, wherein the intensity of the physiological signal (EEG) of the user is determined by estimating a signal-to-noise ratio of the physiological signal (EEG) measured on the user.

14. Method according to Claims 12 and 13 taken in combination, comprising:
- retaining at least the interface signal appropriate to the user that, when output, has caused maximization, at the modulation frequency, of the signal-to-noise ratio of the physiological signal (EEG) measured on the user.

15. Computer program comprising instructions for carrying out the method according to one of the preceding claims when this program is executed by a processing circuit.

16. Device comprising a processing circuit for carrying out the method according to one of Claims 1 to 14.
